# FASCICULE DE BREVET EUROPEEN

(11) **EP 0 518 728 B1**
(45) Date de publication et mention de la délivrance du brevet: **18.10.1995**
(21) Numéro de dépôt: 92401501.9
(22) Date de dépôt: 02.06.1992
(51) Int. Cl.: C07C 249/00, C07C 251/88

(54) **Procédé pour réduire la teneur en CO2 dans les réacteurs de synthèse d'azines**
Verfahren zur Reduzierung von CO2-Inhalt in einem Reaktor für Azinen-Synthese
Process for reducing the C02-content in reactors for the synthesis of azines

(30) Priorité: 12.06.1991 FR 9107149
(43) Date de publication de la demande: 16.12.1992
(73) Titulaire: ELF ATOCHEM S.A., 92800 Puteaux (FR)
(72) Inventeur: Krempf, Gérard, F-69003 Lyon (FR); Collier, Bertrand, F-65250 La Barthe de Neste (FR); Tellier, Pierre, F-69110 Saint Foy Les Lyon (FR); Schirmann, Jean-Pierre, F-69600 Oullins (FR)

(56) Documents cités:
- EP-A- 0 399 866
- FR-A- 2 338 252
- US-A- 3 948 902

## Description

La présente invention concerne un procédé pour réduire la teneur en CO₂ dans les réacteurs de synthèse d'azines.

La synthèse de l'hydrazine à partir d'ammoniac et d'eau oxygénée est décrite dans ULLMANN'S ENCYCLOPEDIA OF INDUSTRIAL CHEMISTRY (1989) Vol. A 13, pages 182-183.

Dans une première étape on forme une azine selon la réaction : 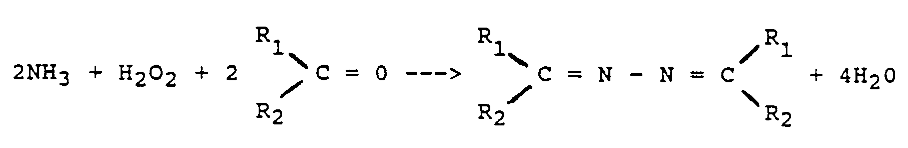
Cette réaction s'effectue en présence d'un catalyseur ou d'un mélange de catalyseurs. Ce catalyseur est utilisé sous forme d'une solution de travail. En fin de réaction on sépare l'azine de la solution de travail.

La solution de travail est régénérée puis recyclée à cette première étape. Dans une deuxième étape l'azine est hydrolysée en hydrazine, la cétone récupérée est recyclée à la première étape : 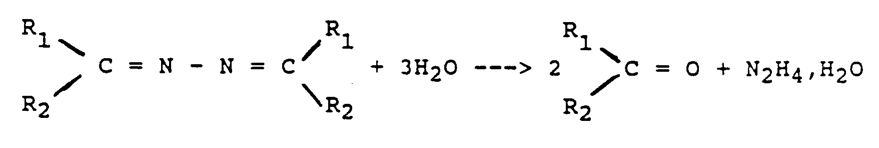
Ce procédé a été décrit dans les brevets US 3972878, US 3972876, US 3869541, US 3948902, US 3956282, US 3943152, US 4725421, US 4093656, US 4724133 et EP 399866.

Dans la première étape on effectue l'alimentation des réactifs NH₃ et H₂O₂.

On a maintenant découvert qu'il fallait éviter autant que possible la présence de CO₂ pendant la réaction de synthèse d'azines. Par exemple en effectuant la synthèse d'azines par réaction de la méthyléthylcétone, de NH₃ et de l'eau oxygénée en présence d'une solution de travail qui est une solution aqueuse d'acétamide, d'acétate de sodium et de phosphate disodique. On a observé que la perte d'eau oxygénée par décomposition augmentait avec la concentration en CO₂ du milieu réactionnel c'est-à-dire de l'ensemble solution de travail et réactifs.

La présente invention est donc un procédé de synthèse d'azines par mise en contact d'ammoniac, d'eau oxygénée et d'un réactif portant un groupe carbonyle avec une solution de travail contenant un catalyseur pour transformer ces réactifs en azine caractérisé en ce qu'on opère en l'absence de CO₂.

Cette réaction est connue en soi, elle a été décrite dans les brevets cités précédemment. Le réactif portant un groupe carbonyle est un aldéhyde ou une cétone. On utilise le plus souvent l'acétone, la méthyléthylcétone (MEK) la méthylisobutylcétone ou la cyclohéxanone. La solution de travail peut être toute solution capable de convertir un mélange d'ammoniac d'eau oxygénée et d'un réactif portant un groupe carbonyl en une azine. C'est par exemple une solution aqueuse d'acétate d'ammonium et d'acétamide.

"En l'absence de CO₂" veut dire qu'on opère avec le moins possible de CO₂ c'est-à-dire sans introduire de CO₂ dans le réacteur et que l'ammoniac, l'eau oxygénée, le réactif portant un groupe carbonyl, la solution de travail, les recyclages éventuels d'ammoniac et en général toute entrée dans le réacteur ne contient pas de CO₂. Il se peut qu'il se forme du CO₂ dans le réacteur, mais l'invention consiste à opérer avec des courants d'entrée dans le réacteur qui ne contiennent pas ou peu de CO₂. Ce résultat est d'autant plus surprenant que d'après l'art antérieur FR 2338252 si on place dans un réacteur 65 g (3,6 moles) d'eau, 14,5 g (0,25 mole) d'acétone, 5 g (0,1 mole) de solution aqueuse de péroxyde d'hydrogène à 68 % en poids, 0,25 g de sel disodique de l'acide éthylène diaminététracétique, puis qu'on fait barboter une courant de NH₃ ainsi qu'un courant de CO₂ à 25°; après 24 heures on dose dans le mélange 4,12 g d'azine de l'acétone (0,037 mole), ce qui correspond à un rendement de 36,8 % par rapport au péroxyde d'hydrogène.

Le sel disodique de l'éthylène diamine tétracétique est un séquestrant des ions lourds et ainsi stabilise l'eau oxygénée.

Selon ce même art antérieur si on reproduit cet exemple sans introduire de CO₂ on ne trouve pas de trace d'azine. Le CO₂ peut donc être considéré comme catalyseur puisqu'il permet la production d'azine. Cependant le rendement est de 36,8 %, alors qu'en utilisant un mélange d'acétate d'ammonium, d'acétamide et de phosphate disodique on obtient des rendements supérieurs à 85 %.

L'invention concerne aussi un procédé de synthèse d'azines dans lequel :
a) on met en contact de l'ammoniac, de l'eau oxygénée et un réactif portant un groupe carbonyle avec une solution de travail contenant un catalyseur pour transformer les réactifs en azine
b) on sépare l'azine de la solution de travail
c) on régénère la solution de travail en la portant à au-moins 130°C tout en éliminant l'eau de réaction et l'eau apportée par l'eau de dilution de l'eau oxygénée sous forme d'un courant contenant de l'eau, de l'ammoniac, du réactif portant un groupe carbonyl et du CO₂
d) on recycle la solution de travail à l'étape a caractérisé en ce qu'on recycle le courant de l'étape c vers le réacteur de l'étape a après en avoir éliminé le CO₂ et l'essentiel de l'eau.

On a en effet découvert que bien qu'on n'introduise pas de CO₂ dans l'étape a et que la réaction de synthèse d'azine ne génère pas de CO₂ il s'en forme un peu par dégradation des produits et réactifs sous action de l'eau oxygénée dans l'étape a et pendant la régénération de l'étape c. Si on ne purge pas le CO₂ on observe une dégradation du rendement en eau oxygénée. Dans le courant de l'étape c l'essentiel du CO₂ est sous forme de carbonate d'ammonium.

L'ammoniac, le réactif portant un groupe carbonyle qu'on récupère après avoir éliminé le CO₂ et l'essentiel de l'eau sont recyclés dans le réacteur de synthèse. L'exemple 1 décrit un tel procédé.

On peut utiliser différents procédés pour éliminer le CO₂ et l'essentiel de l'eau du courant de l'étape c; on peut envoyer ce courant en tête d'une colonne à plateaux ou à garnissage qu'on fait fonctionner corne colonne d'épuisement ou de stripping en chauffant le pied de sorte qu'on récupère en tête l'ammoniac, le réactif portant le groupe carbonyle le CO₂ de l'eau et en pied la majeure partie de l'eau. On condense ensuite le courant de tête et on le met sous basse pression à une température entre 20 et 45°C pour retenir le CO₂ sous forme de carbonate d'ammonium en solution aqueuse tandis qu'on trouve en phase gazeuse le réactif portant le groupe carbonyle, l'ammoniac et de la vapeur d'eau. Avantageusement il suffit de se mettre sous une pression absolue entre 4.10³ Pa (30 mmHg) et 16.10³ Pa (120 mmHg) et de préference 8.10³ Pa (60 mmHg) à 12.10³ Pa (90 mmHg).

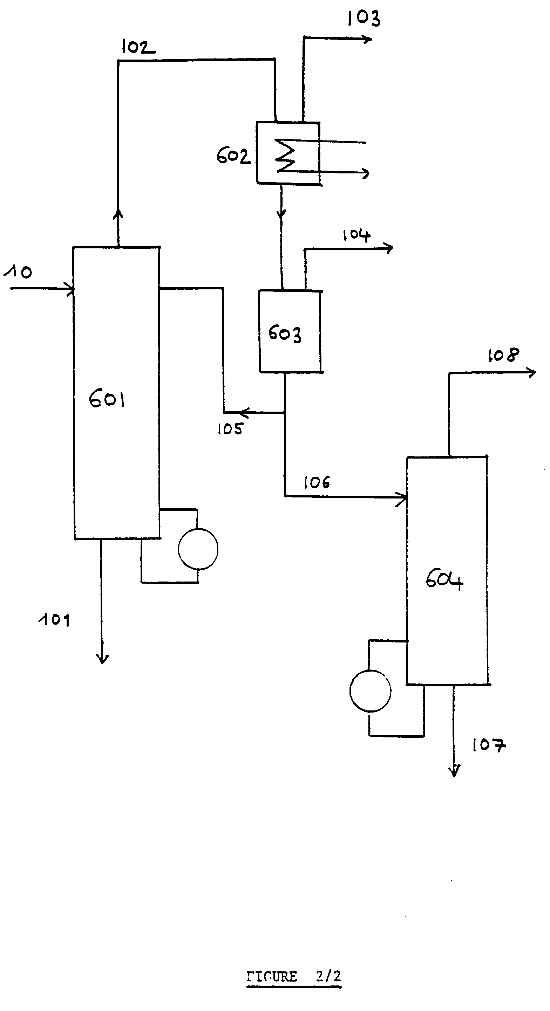

La figure 2 décrit un mode de réalisation possible de l'invention. Le courant 10 qui provient de l'étape c contenant NH₃, le réactif portant un groupe carbonyle du CO₂ et de l'eau arrive en tête d'une colonne de stripping 601 munie d'un rebouilleur. Le CO₂, dans 10, est sous forme de carbonate d'ammonium. On récupère en pied un courant 101 d'eau et un courant de tête 102 qu'on refroidit entre 30 et 50°C dans un condenseur 602. Une partie de NH₃ ne se condense pas et est recylée par un courant 103 au réacteur de synthèse de l'étape a. La phase liquide passe dans le bac 603.

Selon la solubilité du réactif portant un groupe carbonyle on peut avoir une phase organique riche en ce réactif, on la recycle par un courant 104 vers le réacteur de l'étape a. La phase aqueuse contenue dans 603 contient tout le CO₂ sous forme de carbonate d'ammonium qui existait dans le courant 10. Une partie 105 est le reflux de la colonne 601, le courant 106 est flashé sous basse pression dans une colonne 604.

La température de pied est maintenue entre 20 et 45°C par un rebouilleur, la pression est maintenue entre 8.10³ Pa (60 mmHg) et 12.10³ Pa (90 mmHg) absolus par une pompe à vide ou tout dispositif équivalent. Le courant de pied 107 contient tout le CO₂ sous forme de carbonate d'ammonium, de l'eau, des traces de NH₃ et de réactif portant un groupe carbonyle. Le courant 108 de tête contient NH₃, le réactif portant un groupe carbonyle et de l'eau.

En plus des quatre produit du courant 10 qu'on a cités à savoir NH₃, le réactif portant un groupe carbonyle, le CO₂ et l'eau il peut y avoir aussi divers produits organiques tels que l'oxime, de l'azine du butanol secondaire. Le courant 108 est recyclé au réacteur de l'étape a.

On ne sortirait pas du cadre de l'invention en éliminant le CO₂ par d'autres moyens tels qu'un lavage avec la soude pour retenir le CO₂ sous forme de carbonate et laisser l'ammoniac, le réactif portant un groupe carbonyl et la vapeur d'eau sous forme gazeuse.

Avantageusement le réactif portant le groupe carbonyle est choisi comme précédemment parmi les cétones déjà citées. On utilise de préférence la MEK puisque l'azine de la MEK est insoluble dans les solutions aqueuses ce qui facilite l'étape b. Une solution de travail particulièrement simple est constituée d'une solution aqueuse d'acétamide et d'acétate d'ammonium ou d'acétamide et d'acide acétique.

### Exemple 1 (comparatif)

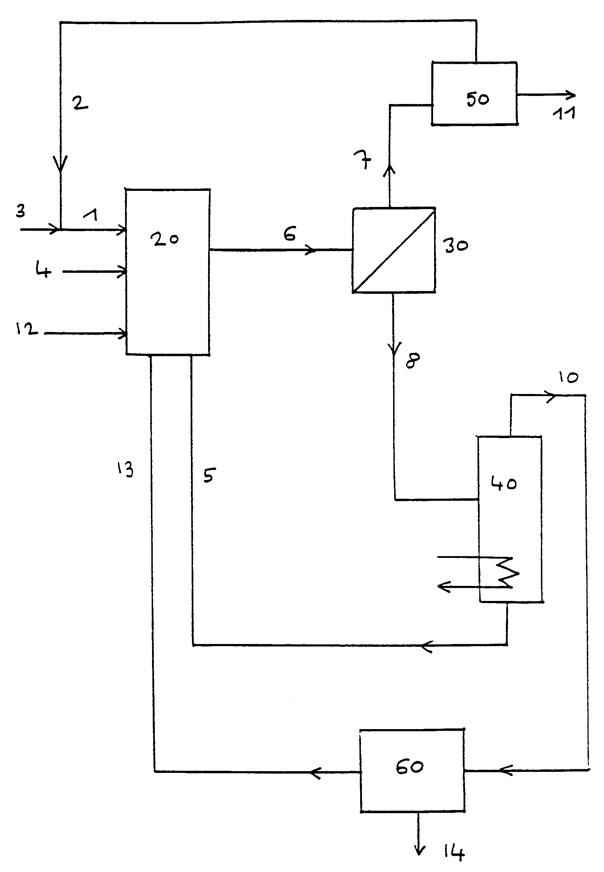

La figure 1 représente un dispositif pour effectuer la synthèse de l'hydrazine à partir d'ammoniac et d'eau oxygénée. Dans le réacteur 20 on effectue la synthèse de l'azine, 1 représente l'alimentation en cétone constituée du recyclage venant du réacteur d'hydrolyse 50 et d'un appoint éventuel 3. 4 représente l'alimentation en NH₃, 12 l'alimentation en eau oxygénée, 5 représente le recyclage de la solution de travail. Par un tuyau 6 le produit de synthèse est amené dans le séparateur 30, qui produit en 7 l'azine brute et en 8 la solution de travail qui contient aussi de l'ammoniac, un peu de cétone, l'eau qui s'est formée par réaction, et l'eau qui a été apportée par l'eau oxygénée puisque pour des raisons de sécurité on utilise l'eau oxygénée à une concentration d'au plus 70 % en poids dans l'eau.

Le séparateur 30 peut être un simple décanteur si l'azine est insoluble dans la solution de travail et dans l'eau de réaction, sinon on utilise une colonne à distiller. La solution de travail ainsi que sa régénération sont décrites dans le brevet EP 399866. La solution de travail a pour fonction de catalyser la synthèse d'azines et d'entraîner l'eau de réaction et l'eau apportée avec l'eau oxygénée par le tuyau 8 jusqu'au dispositif 40. La solution de travail y est portée à au moins 130°C et de préférence entre 150 et 250°C. On récupère en 10 un courant contenant l'eau formée par la réaction et l'eau apportée par l'eau oxygénée de l'ammoniac, de la cétone et en 5 la solution de travail régénérée qu'on renvoie au réacteur 20. On peut éventuellement disposer sur le courant 5 un système pour purifier la solution de travail de ses impuretés lourdes, en effet il se produit inévitablement des dégradations des produits et des réactifs dans le réacteur 20.

En 50 l'azine est hydrolysée en hydrazine qu'on soutire en 11 et en 2 on recycle la cétone au réacteur 20. En régime stationnaire on retrouve en 10 l'eau de dilution de l'eau oxygénée, l'eau formée par la réaction, de l'ammoniac et de la cétone. La synthèse d'azine produit 4 moles d'eau, on retrouve en 10 ces 4 moles et l'eau de dilution de l'eau oxygénée. Une partie de cette eau peut être utilisée en 50 c'est-à-dire 3 moles.

Le bilan eau est excédentaire mais avant de purger cette eau il faut récupérer l'ammoniac et la cétone contenues et les renvoyer au réacteur 20. Il n'est pas souhaitable d'utiliser pour l'hydrolyse en 30 une eau chargée en ammoniac. Pour exploiter économiquement ce procédé il est donc avantageux de récupérer l'ammoniac et la cétone contenues dans le courant 10 et de les renvoyer au réacteur 20.

La solution 10 est traitée en 60 ; on obtient un courant 14 d'eau épurée et en 13 un courant constitué d'eau, d'ammoniac et de cétone qu'on recycle au réacteur 20. La demanderesse a constaté qu'en supprimant ce recyclage 13 on observait à production constante d'azine une consommation supplémentaire d'ammoniac et de cétone correspondant aux quantités qu'on ne recyclait plus par 13 mais aussi un gain sur la consommation d'eau oxygénée. La demanderesse a ainsi découvert que le courant 13 contenait du CO₂ et qu'il provoquait une surconsommation d'eau oxygénée. En coupant le recyclage 13 et en injectant dans le réacteur 20 du CO₂ on observe une surconsommation d'eau oxygénée.

### Exemple 2

On effectue une synthèse d'azines par réaction de la méthyléthylcétone (MEK), NH₃ et l'eau oxygénée en présence d'une solution de travail à base d'acétamide et d'acétate d'ammonium on obtient dans l'étape c un courant 10 contenant de la MEK, NH₃, CO₂, H₂O un peu de butanol secondaire de l'oxime et de l'azine.

On effectue l'élimination du CO₂ et de l'essentiel de l'eau selon le schéma de la figure 2. Les résultats pour deux allures de marche sont reportés sur les tableaux 1 et 2 suivants.
La pression de la colonne 604 est de 12.10³ Pa (90 mmHg) absolus, la température de pied : 30°C.

**TABLEAU 1**

| | **Courant 106** | | **Courant 107** | | **Courant 108** | |
|---|---|---|---|---|---|---|
| | % | kg/h | % | kg/h | % | kg/h |
| CO₂ | 1,1 | 6,4 | 1,973 | 6,4 | 0 | 0 |
| NH₃ | 18,53 | 107,5 | 9,610 | 31,175 | 29,86 | 76,325 |
| MEK | 17,06 | 99 | 4,273 | 13,86 | 33,308 | 85,14 |
| BuOH₂ | 8,88 | 51,5 | 4,531 | 14,7 | 14,396 | 36,8 |
| Azine | 0,62 | 3,6 | 0,709 | 2,3 | 0,508 | 1,3 |
| Oxime | 1,55 | 9,0 | 2,775 | 9 | env. 0 | env. 0 |
| Divers | 1,38 | 8 | 1,54 | 5 | 1,174 | 3 |
| H₂O | 50,862 | 295 | 74,587 | 241,95 | 20,753 | 53,05 |
| **TOTAL** | **99,98** | **580** | **99,998** | **324,385** | **99,998** | **255,615** |

**TABLEAU 2**

| | **Courant 106** | | **Courant 107** | | **Courant 108** | |
|---|---|---|---|---|---|---|
| | % | kg/h | % | kg/h | % | kg/h |
| CO₂ | 0,61 | 1,507 | 1,276 | 1,507 | 0 | 0 |
| NH₃ | 19,25 | 47,547 | 4,43 | 5,23 | 32,66 | 42,31 |
| MEK | 22,3 | 55,08 | 4,66 | 5,5 | 38,26 | 49,572 |
| BuOH₂ | 2,5 | 6,175 | 1,06 | 1,255 | 3,8 | 4,92 |
| Azine | 0,25 | 0,6175 | 0,33 | 0,3875 | 0,177 | 0,23 |
| Oxime | 1,02 | 2,519 | 1,58 | 1,865 | 0,504 | 0,654 |
| Divers | 5,74 | 14,178 | 8,38 | 9,903 | 3,3 | 4,275 |
| H₂O | 48,58 | 120 | 78,26 | 92,4 | 21,3 | 27,6 |
| **TOTAL** | **100** | **247,62** | **99,98** | **118,062** | **100** | **129,56** |

## Revendications

1. Procédé de synthèse d'azines en l'abscence de CO₂, par mise en contact d'ammoniac, d'eau oxygénée et d'un réactif portant un groupe carbonyle choisi parmi un aldéhyde et une cétone avec une solution de travail contenant un catalyseur pour transformer ces réactifs en azine caractérisé en ce que dans ledit procéde aucun CO₂ est introduit et le CO₂ de réaction est purgé.

2. Procédé de synthèse d'azines dans selon la revendication 1 lequel :
a) on met en contact de l'ammoniac, de l'eau oxygénée et un réactif portant un groupe carbonyle avec une solution de travail contenant un catalyseur pour transformer les réactifs en azine ;
b) on sépare l'azine de la solution de travail ;
c) on régénère la solution de travail en la portant à au moins 130°C tout en éliminant l'eau de réaction et l'eau apportée par l'eau de dilution de l'eau oxygénée sous forme d'un courant contenant de l'eau de l'ammoniac du réactif portant un groupe carbonyle et du CO₂ de réaction.
d) on recycle la solution de travail à l'étape a caractérisé en ce qu'on recycle le courant de l'étape c vers le réacteur de l'étape a après en avoir éliminé le CO2 et l'essentiel de l'eau.

3. Procédé selon la revendication 2 caractérisé en ce que pour éliminer le CO₂ et l'essentiel de l'eau du courant venant de l'étape c :
- on fait passer ce courant dans une colonne de stripping, l'eau étant éliminée en pied ;
- on condense le courant de tête et on le met sous basse pression à une température comprise entre 20 et 45°C pour obtenir en pied un courant essentiellement de carbonate d'ammonium et en tête un courant d'ammoniac, du réactif portant le groupe carbonyle et de l'eau.

4. Procédé selon la revendication 3 caractérisé en ce que la basse pression est comprise entre 4.10³ Pa (30 mmHg) et 16.10³ Pa (120 mmHg) absolus et de préférence 8.10³ Pa (60 mmHg) à 12.10³ Pa (90 mmHg).

5. Procédé selon l'une des revendications 1 à 4 caractérisé en ce que la cétone est choisie parmi l'acétone, la méthyléthylcétone, la méthylisobutylcétone et la cyclohexanone.

6. Procédé selon l'une des revendications 1 à 5 caractérisé en ce que la solution de travail est une solution aqueuse d'acétamide et d'acétate d'ammonium ou d'acétamide et d'acide acétique.

## Claims

1. Process for the synthesis of azines in the absence of CO₂ by bringing ammonia, aqueous hydrogen peroxide and a reactant carrying a carbonyl group chosen from an aldehyde and a ketone into contact with a working solution containing a catalyst for converting these reactants into azine, characterized in that in the said process, no CO₂ is introduced, and the CO₂ of reaction is purged.

2. Process for the synthesis of azines according to Claim 1, in which:
a) ammonia, aqueous hydrogen peroxide and a reactant carrying a carbonyl group are brought into contact with a working solution containing a catalyst for converting the reactants into azine;
b) the azine is separated from the working solution;
c) the working solution is regenerated by being taken to at least 130°C whilst removing the water of reaction and the water introduced by the water of dilution of the aqueous hydrogen peroxide in the form of a stream containing water, ammonia, the reactant carrying a carbonyl group and CO₂ of reaction;
d) the working solution is recycled to stage a,
characterized in that the stream from stage c is recycled towards the reactor of stage a after the CO₂ and most of the water have been removed therefrom.

3. Process according to Claim 2, characterized in that, in order to remove the CO₂ and most of the water from the stream originating from stage c:
- this stream is passed through a stripping column, the water being removed at the bottom;
- the top stream is condensed and is placed under low pressure at a temperature of between 20 and 45°C to obtain at the bottom a stream essentially of ammonium carbonate and at the top a stream of ammonia, the reactant carrying the carbonyl group and water.

4. Process according to Claim 3, characterized in that the low pressure is between 4×10³Pa (30 mm Hg) and 16×10³Pa (120 mm Hg) absolute and preferably 8×10³Pa (60 mm hg) to 12×10³Pa (90 mm Hg).

5. Process according to one of Claims 1 to 4, characterized in that the ketone is chosen from acetone, methyl ethyl ketone, methyl isobutyl ketone and cyclohexanone.

6. Process according to one of Claims 1 to 5, characterized in that the working solution is an aqueous solution of acetamide and ammonium acetate or of acetamide and acetic acid.

## Patentansprüche

1. Verfahren zur Azinsynthese in Abwesenheit von CO₂, durch Inkontaktbringen von Ammoniak, Wasserstoffperoxid und einem eine Carbonylgruppe tragenden Reagenz, das aus Aldehyden und Ketonen ausgewählt ist, mit einer Arbeitslösung, enthaltend einen Katalysator zur Umwandlung dieser Reagenzien in Azin, dadurch gekennzeichnet, daß bei dem genannten Verfahren kein CO₂ hinzugegeben wird und das aus der Reaktion stammende CO₂ entfernt wird.

2. Verfahren zur Azinsynthese nach Anspruch 1, in dem man:
a) Ammoniak, Wasserstoffperoxid und ein eine Carbonylgruppe tragendes Reagenz mit einer einen Katalysator enthaltenden Arbeitslösung zur Umwandlung der Reagenzien in Azin in Kontakt bringt,
b) das Azin von der Arbeitslösung trennt,
c) die Arbeitslösung regeneriert, indem sie auf mindestens 130 °C erhitzt wird, wobei das Reaktionswasser und das zum Verdünnen des Wasserstoffperoxids zugesetzte Wasser in Form eines Stroms entfernt werden, der Wasser, Ammoniak, das eine Carbonylgruppe enthaltende Reagenz und das aus der Reaktion stammende CO₂ enthält,
d) die Arbeitslösung zum Schritt a) rückfuhrt,
dadurch gekennzeichnet, daß man den Strom aus Schritt c) in den Reaktor des Schrittes a) zurückleitet, nachdem das CO₂ und der größte Teil des Wassers daraus entfernt wurden.

3. Verfahren nach Anspruch 2, dadurch gekennzeichnet, daß man zur Entfernung des CO₂ und des größten Teils des Wassers aus dem Strom des Schritts c):
- diesen Strom durch eine Stripping-Kolonne leitet, wobei das Wasser am Kolonnenboden entfernt wird;
- den Gasstrom des Kolonnenkopfes kondensiert und einem reduzierten Druck bei einer Temperatur zwischen 20 und 45 °C aussetzt, um am Kolonnenboden einen im wesentlichen aus Ammoniumcarbonat bestehenden Strom und am Kolonnenkopf einen Strom aus Ammoniak, dem die Carbonylgruppe enthaltenden Reagenz und Wasser zu erhalten.

4. Verfahren nach Anspruch 3, dadurch gekennzeichnet, daß der reduzierte Druck zwischen 4·10³ Pa (30 mmHg) und 16·10³ Pa (120 mmHg), vorzugsweise zwischen 8·10³ Pa (60 mmHg) und 12·10³ Pa (90 mmHg), liegt.

5. Verfahren nach einem der Ansprüche 1 bis 4, dadurch gekennzeichnet, daß das Keton aus Aceton, Methylethylketon, Methylisobutylketon und Cyclohexanon ausgewählt ist.

6. Verfahren nach einem der Ansprüche 1 bis 5, dadurch gekennzeichnet, daß die Arbeitslösung eine wäßrige Lösung aus Acetamid und Ammoniumacetat oder Acetamid und Essigsäure ist.
